# EUROPEAN PATENT SPECIFICATION

(11) **EP 1 798 556 B1**
(45) Date of publication and mention of the grant of the patent: **26.08.2009**
(21) Application number: 05027345.7
(22) Date of filing: 14.12.2005
(51) Int. Cl.: G01N 33/569, C07K 14/16

(54) **Device for the early and rapid immunochromatographic detection of HIV and uses thereof**
Vorrichtung zum frühen und schnellen immunochromatographischen Nachweis von HIV und dessen Anwendung
Dispositif pour la détection immunochromatographique précoce et rapide de l HIV, et usages correspondants

(43) Date of publication of application: 20.06.2007
(73) Proprietor: The Jordanian Pharmaceutical Manufacturing Co., 11710 Naor (JO)
(72) Inventor: Badwan, Adnan, Dr., Amman 11185 (JO); Mohammed, Murshed Abdel-Qader Murshed, Amman 11118 (JO); El-Taher, Tala Saleh Hamdan, Dr., Amman 11953 (JO)
(74) Representative: Winkler, Andreas Fritz Ernst

(56) References cited:
- WO-A-93/21346
- CA-A1- 2 431 778
- GB-A- 2 313 666
- US-A- 5 830 641
- US-A- 6 146 589
- US-A1- 2004 072 267
- US-B1- 6 653 066
- HUANG HAIZHEN ET AL: "Synthesis of chitosan-stabilized gold nanoparticles in the absence/presence of tripolyphosphate" BIOMACROMOLECULES, vol. 5, no. 6, November 2004 (2004-11), pages 2340-2346, XP002379577 ISSN: 1525-7797

## Description

This invention provides a device for the simple and rapid detection of HIV in a sample that allows the detection of early human HIV infection. The device comprises a rapid immunochromatographic test for ultra sensitive p24 antigen detection. The device furthermore enables to distinguish between HIV-1 and HIV-2 infection. The invention further provides uses of the device in a method as well as a kit for the rapid detection of HIV in a sample.

### Background of the invention

The number of people living with HIV has now risen to reach its highest level ever: close to 40 million people are living with the virus and close to 5 million people were newly infected with HIV in 2004 alone. Worldwide, the AIDS epidemic killed over 3 million people last year alone (Source: UNAIDS). Furthermore, only one in five people needing HIV prevention worldwide have access to basic prevention services and only one in ten people living with HIV has been tested for the virus.

The HI virus is most easily transmitted to others during the initial period of acute HIV infection, when the viral load (quantity of HIV RNA in the blood) is especially high and when people are not aware of being contaminated by the virus. Most HIV infections are transmitted at this stage, called primary infection. Earlier detection using ultra sensitive tests avoids missing primary infections, enabling immediate precautionary measures to be taken to help prevent the risk of HIV transmission to a non-infected partner, to an unborn child, or through blood donations or direct blood contact. Earlier detection of HIV infection also ensures the implementation of early antiretroviral therapy (ART) to slow down the progression of HIV infection, thereby improving patient care and quality of life.

The diagnosis of HIV infection is usually made on the basis of the detection of HIV antibodies and/or antigen. The diagnosis of an HIV infection can be made indirectly, i.e. through the demonstration of virus-specific antibodies. Besides such indirect diagnosis based on detection of antibodies, a direct diagnosis of HIV infection is also possible: either through the demonstration of infectious virus (using cell culture), viral antigens (p24 antigen ELISA) or viral nucleic acid (i.e. viral genome); the latter is also termed nucleic acid testing (NAT).

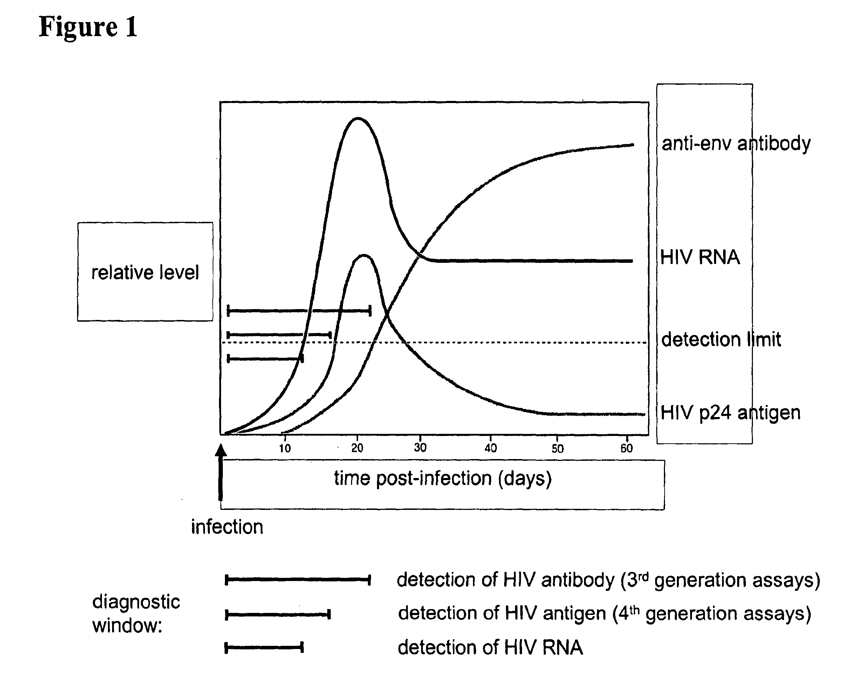

One important problem of HIV antibody testing is the so-called "diagnostic window". This is the time period that elapses between the time of acquisition of HIV infection until detectable levels of antibodies are present (see Figure 1). The switch from antibody-negative to antibody-positive is called "seroconversion".

The most widely used screening tests are ELISAs as they are the most appropriate for screening large numbers of specimens on a daily basis, e.g. blood donations. The earliest assays used purified HIV lysates (1^{st} generation assays). Improved assays based on recombinant proteins and/or synthetic peptides, which also enabled the production of combined HIV-1/HIV-2 assays, became rapidly available (2^{nd} generation assays). The so-called 3^{rd} generation or antigen-sandwich assays, which use labeled antigens as conjugate, are more sensitive and have reduced the diagnostic window period considerably [1-2] (see Figure 1).

The window interval between the presence of HIV-1 RNA in plasma and antibody seroconversion varies between 27.4 and 10.2 days depending on the route of infection. HIV infection is detected between 17.4 and 9.4 days earlier by testing for the HIV antigen p24 compared to the above mentioned 3^{rd} generation assays [3], see also Figure 1. Testing for the antigen p24 was the first assay available for the diagnosis of HIV infection prior to antibody seroconversion and has been available commercially since 1986 for the detection of HIV antigen in serum, plasma and cerebrospinal fluid (CSF) using enzyme immunoassay (EIA) technology [4-7].

Although the prevalence and incidence of HIV infection in the general population in industrialized countries are relatively low, the residual risk for HIV transmission by blood donation (mostly by viremic but antibody negative donors) is ~ 1/493 000 per unit in the USA [8]. Despite the efforts to improve self-exclusion, some donors may not report or not perceive risk behavior for HIV infection [9]. By additional screening for the p24 antigen, the risk of HIV infection may be reduced to 1/676 000. However, the cost-effectiveness of such testing is predicted to be far below that of most medical interventions [10]. In countries with high transmission rates, i.e. Thailand where the H IV incidence is 10-100 times than that in the USA, p24 antigen testing of donors has been shown to be highly cost-effective [11].

Furthermore, anti-retroviral therapy is becoming available more readily throughout the developing world as a result of increases in health budgets as well as drastic price reductions negotiated with pharmaceutical companies. Unfortunately, the cost of serological tests required for the follow up of the treatment remains highly prohibitive for the majority of the patients in resource-limited countries. This situation may not only contribute to render anti-retroviral therapy ineffective but also contribute to the emergence of drug-resistant HIV strains [12]. A single PCR-based determination of the RNA viral load can cost US$ 100 or more per test, which is equal to the costs for one month of life-saving treatment for an HIV patient receiving anti-retroviral therapy [13]. The development and implementation of reliable low-cost tools to monitor the effectiveness of treatment is therefore urgently desired.

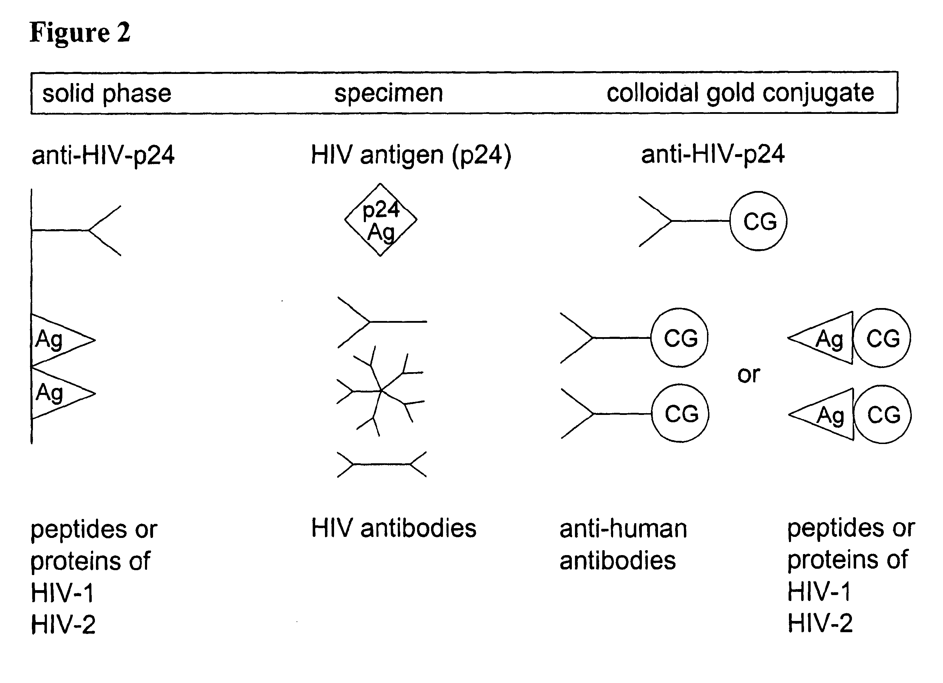

In recent years, the development of enhanced ELISA assays that detect both HIV antibody and antigen (4^{th} generation assays, see Figure 2A) has led to earlier detection of HIV seroconversion by further reducing the diagnostic window period. 4^{th} generation HIV assays allow the combined detection of HIV antigen and antibody (Ag/Ab) in order to reduce the diagnostic window between infection and antibody detection in primary HIV infection. These 4^{th} generation HIV screening assays are known in the art since the late 1990s. These assays are reported to reduce the diagnostic window of HIV infection by an average of 7 days in comparison to the 3^{rd} generation HIV screening assays. Therefore, an earlier diagnosis of HIV infection is possible, by detecting p24 antigen which may be present in samples from individuals with recent HIV infection prior to seroconversion.

Several 4^{th} generation HIV screening assays are also commercially available [14]. For example, AxSYM® HIV Ag-Ab (Abbott Laboratories, Abbott Park, USA), Murex HIV Ag/Ab Combination (Abbott/Murex Biotech Ltd., Dartford, UK), Genscreen® Plus HIV Ag-Ab (Bio-Rad Laboratories, Hercules, USA) and Enzymun-Test® HIV Combi (Roche Boehringer Mannheim, Penzberg, Germany). In these commercial assays, the presence of HIV antibodies and p24 antigen is detected at the same time as one combined signal, However, such one combined signal does not allow to discriminate whether the positive signal is based on a detection of antibodies or antigen or both. There further exist for example Vironostika® HIV Uni-Form II Ag/Ab (Organon Teknika, Boxtel, Netherlands) or Enzygnost® HIV Integral (Dade Behring, Mannheim, Germany).

Furthermore, there are the commercially available 4^{th} generation assays of the type VIDAS® HIV DUO, such as VIDAS® HIV DUO and VIDAS® HIV DUO ULTRA (bioMérieux, Lyon, France). They utilize solid-phase receptacles, divided in a lower and an upper part, wherein the lower part detects HIV antibodies and the upper part detects HIV p24, i.e. the presence of HIV antibodies and p24 antigen can be detected as two separate signals. However, the majority of the commercially available 4^{th} generation HIV screening assays still utilize coated microtiter plates or blends of microparticles (except for assays of the type VIDAS® HIV DUO which utilize solid-phase receptacles).

Recently, a variety of simple, instrument-free initial HIV tests have become available, including agglutination, immunofiltration (flow through tests), immunochromatographic (lateral flow tests) and dipstick tests, which are primarily based on the principle of the 3^{rd} generation assays [15]. Specimen and reagents are often added by means of a dropper to the test device. A positive result is indicated by the appearance of a colored dot or line, or shows an agglutination pattern. Most of these tests can be performed in less than 20 minutes, and are therefore called simple/rapid assays. The results are read visually. Examples for commercially available simple and/or rapid 3^{rd} generation assays are *Instant*CHEK™-HIV 1+2 (EY Laboratories Inc.), GENIE II HIV-1/HIV-2 (Bio-Rad), Efoora HIV Rapid (Efoora Inc.). OraQuick HIV-1/2 Rapid HIV -1/2 antibody (OraSure Technologies Inc.), SD Bioline HIV 1/2 3.0 (Standard Diagnostics Inc.), Hema.Strip® HIV 1/2 (Chembio Diagnostics), HIV 1/2 STAT-PAK (Chembio Diagnostics), HIV (1+2) Antibody (Colloidal Gold) (KHB Shanghai Kehua Bio-engineering Co. Ltd.), GENEDIA® HIV 1/2 Rapid 3.0 (Green Cross Life Science Corp.), DoubleCheckGold™ HIV 1&2 (Orgenics Ltd.), and Uni-Gold™ HIV1/2 (Trinity Biotech Ltd., Ireland) [15]. In general, these simple/rapid tests are most suitable in testing and counselling centres and laboratories that have limited facilities and process low numbers of specimens daily.

Canadian patent application CA 2,431,778 discloses a rapid diagnostic immunoassay that belongs to the 4^{th} generation assays since it allows the combined detection of HIV 1+2 antibodies and the HIV antigen p24. This immunoassay comprises a test strip having an upstream and a downstream end, two test zones and one control zone and a housing having opening and/or transparent materials. One of the two test zones of the test strip contains HIV1+2 antigen for the detection of HIV 1+2 antibodies and the other of the two test zones contains a monoclonal antibody against p24 for the detection of p24 antigen.

However, most commercially available 4^{th} generation HIV screening assays reduce but still cannot close the diagnostic window during primary HIV infection. Therefore, HIV screening assays always need to be judged in the context of symptoms and risk factors. Furthermore, the sensitivity of the p24 antigen detection in 4^{th} generation assays needs to be improved [16]. Therefore, there is a need for further improving 4^{th} generation assays in order to increase the sensitivity as well as the specificity.

Therefore, the problem to be solved by this invention was to provide more sensitive device which allows the detection of early human HIV infection.

The problem is solved by a device having the features of claim 1.

The test device according to the invention comprises a housing (1) which comprises two test strips (2, 3), wherein each test strip comprises at least one sample application site (21, 31), at least one test zone (22a, 22b, 32) and one control zone (23, 33).

The housing (1) further comprises openings and/or transparent materials. The openings and/or transparent materials of the housing (1) preferably serve for receiving test samples and for reading the results from the test zones (22a, 22b, 32) and control zones (23, 33).

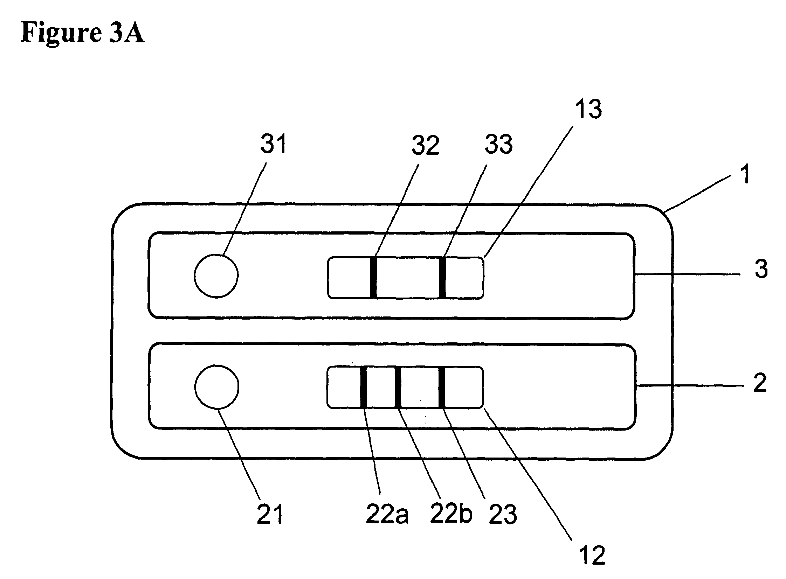

A preferred embodiment is shown in Figure 3A, wherein the two test strips (2, 3) are arranged parallel to each other with the at least one sample application site (21, 31), at least one test zone (22a, 22b, 32) and one control zone (23, 33) also parallel to each other.

Preferred openings of the housing are at the respective at least one sample application site (21, 31) of each test strip (2, 3) to allow for sample application. Preferred openings of the housing can be closed after opening.

A preferred transparent material of the housing will allow for reading the results from the test zones (22a, 22b, 32) and control zones (23, 33) of each test strip (2, 3). In Figure 3A such a preferred transparent material of the housing is shown by reference numbers 12 and 13. The transparent materials of the housing can also have enclosures which can be opened at the time point of reading.

The housing can also completely be made of transparent material.

Preferred materials for the test strips are known in the art, such as nitrocellulose membrane, absorbent cellulose pads, blood filter or wicks. The test strip can further comprise a backing layer, such as a polyvinyl backing layer. The materials of the test strips, such as the nitrocellulose membrane and/or absorbent pads can be assembled onto and connected to the backing layer by the means of an adhesive, preferably a pressure sensitive adhesive.

In a preferred embodiment the sample application site (21, 31) is a sample pad or a sample wick.

The first test strip (2) of the device according to the invention comprises a first test zone (22a) comprising HIV-1 antigen(s) and a second test zone (22b) comprising HIV-2 antigen(s), whereas the second test strip (3) of the device according to the invention comprises a third test zone (32) comprising anti-p24 antibody.

Preferred HIV antigens comprised in the first and second test zones (22a, 22b) are selected from synthetic or recombinant HIV-1 or HIV-2 proteins, respectively, and are preferably selected from p24 and p41 for HIV-1 and p36 for HIV-2. Other HIV proteins like p17, p120, p160 or others are suitable as well. The HIV antigens are preferably immobilized in the respective zone (22a, 22b).

Preferred anti-p24 antibodies comprised in the third test zone (32) are selected from monoclonal or polyclonal antibodies. Even though monoclonal antibodies are more suitable for their higher specificity, polyclonal antibodies can be used as well. The anti-p24 antibodies are preferably immobilized in the respective zone (32)

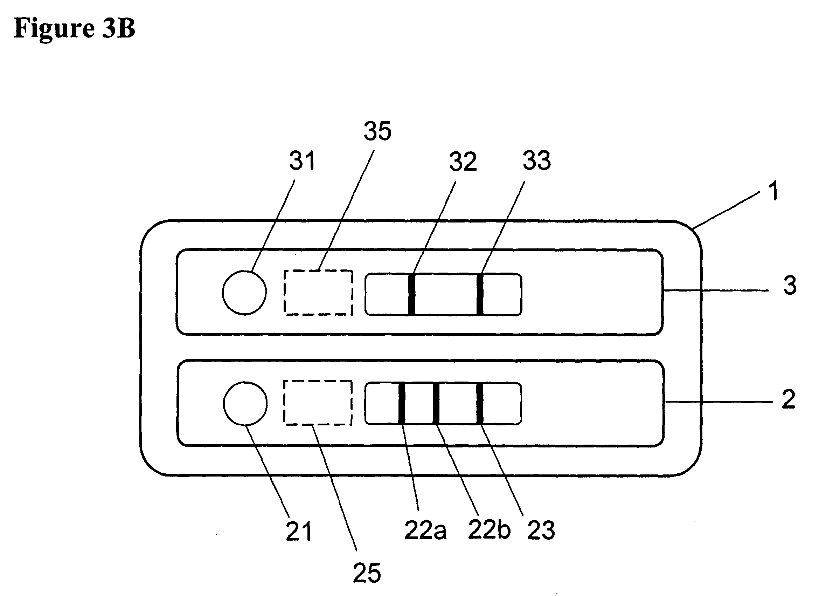

According to the invention, one or both of the two test strips further comprise between the sample application site (21, 31) and the test zones (22a, 22b, 32) a zone comprising gold protein-conjugates (25, 35) (for an embodiment, see Figure 3B). The protein-conjugates can be the same or different to the respective proteins or protein conjugates contained in the respective test zones of the test strips, i.e. synthetic or recombinant HIV-1 or HIV-2 proteins in the first test strip or monoclonal or polyclonal anti-p24 antibodies in the second test strip. The protein-conjugates in zone (25, 35) will preferably be released when liquid, such as the sample, flows through the zone.

A device is preferred wherein said zone (25) is situated in the first test strip (2). It is preferred that the gold protein-conjugates of said zone are gold conjugates of HIV-1 antigens and HIV-2 antigens or gold conjugates of anti-human immunoglobulin(s) or of any protein or molecule that could capture anti-HIV1/2 human antibodies.

A device is preferred wherein said zone (35) is situated in the second test strip (3). It is preferred that the gold protein-conjugates of said zone are polyclonal or monoclonal anti-p24 antibody gold conjugates or any protein or molecule that could capture p24 antigen.

In a preferred embodiment, each test strip comprises said zone comprising gold protein-conjugates.

Furthermore, it is preferred that both test strips (2, 3) comprise said zone comprising protein-conjugates (25, 35), wherein the protein-conjugates comprise the respective gold protein-conjugates, i.e. anti-human immunoglobulin(s) gold conjugates, HIV-1 antigen(s) gold conjugates and HIV-2 antigen(s) gold conjugates or anti-p24 antibody gold conjugates.

In a preferred embodiment the zone comprising protein-conjugates (25, 35) is a fiber glass gold releasing pad, which releases the gold protein-conjugates when liquid, e.g. the sample, flows through it.

It is further preferred that the control zone (23, 33) of each of the two test strips comprise non-specific capturing antibodies, which are preferably immobilized. Preferred non-specific capturing antibodies are antibodies that capture the conjugated protein non-specifically, anti-mouse antibodies in case mouse c lone conjugates were u sed, a nti-rabbit antibodies i n c ase rabbit clones were used. Other preferred capturing antibodies are anti-(anti-human immunoglobulin) control antibodies. Furthermore, in case of using mouse anti-human immunoglobulin colloidal gold conjugate the control antibody will be anti-mouse immunoglobulin. Furthermore, the control zone (23, 33) of each of the two test strips can also comprise human immunoglobulin(s) or human anti-HIV-1/human anti-HIV-2 antigens.

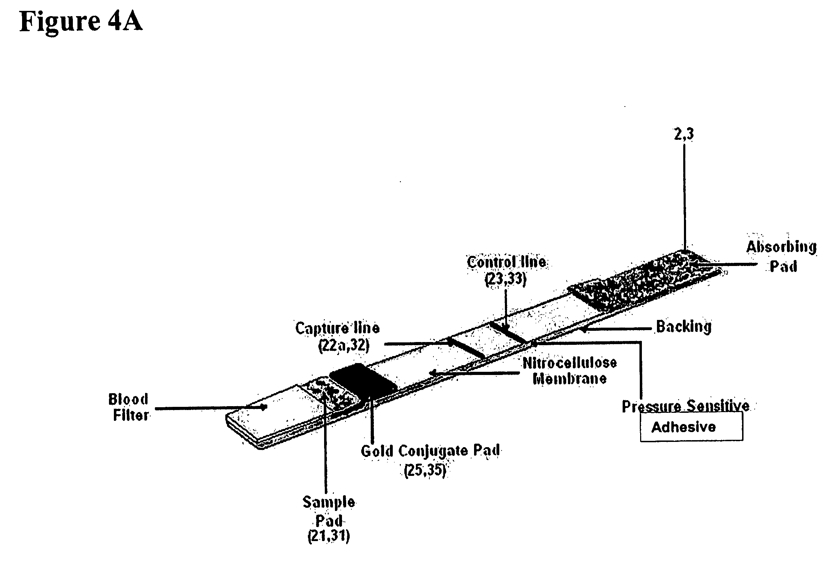

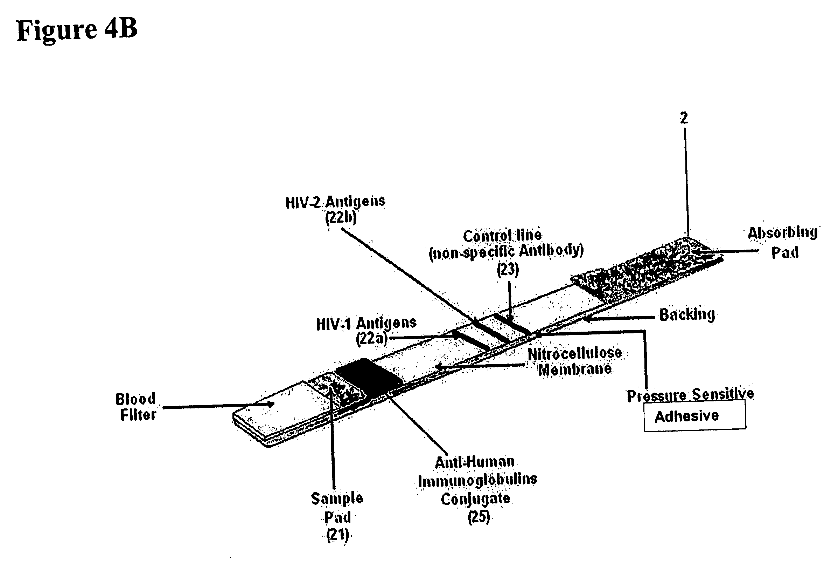

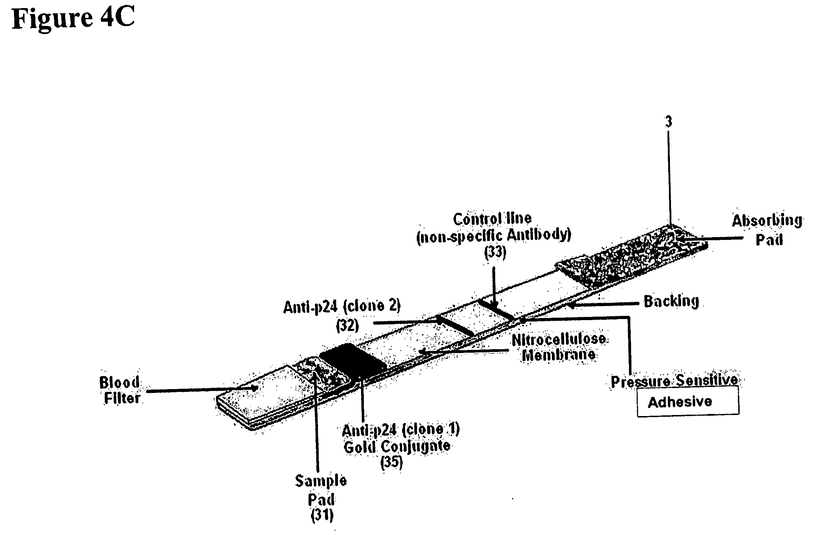

Preferred embodiments of the test strips of the device of the invention are shown in Figure 4.

A preferred test strip (2, 3), as shown in Figure 4A, comprises:
- a backing layer,
- a sample pad as sample application site (21, 31),
- a gold conjugate pad comprising gold protein-conjugates (25, 35),
- a capture line as test zone (22a, 32),
- a control line as control zone (23, 33),
- the material of the test strip in the areas of zones 22a, 32 and 23, 33 is a nitrocellulose membrane,
- the remaining material of the test strip is an absorbing pad and a blood filter, and
- a pressure sensitive adhesive to assemble/connect the sample pad, nitrocellulose membrane, blood filter and absorbing pad to the backing layer.

A preferred first test strip (2), as shown in Figure 4B, comprises
- a backing layer,
- a sample pad as sample application site (21),
- the zone comprising protein-conjugates (25) comprises anti-human immunoglobulins,
- test zones (22a, 22b) comprising immobilized HIV-1 antigen(s) or HIV-2 antigen, respectively,
- a control line with immobilized non-specific antibody as control zone (23),
- the material of the test strip in the areas of zones 22a, 22b and 23 is a nitrocellulose membrane,
- the remaining material of the test strip is an absorbing pad and a blood filter, and
- a pressure sensitive adhesive to assemble/connect the sample pad, nitrocellulose membrane and absorbing pad to the backing layer.

A preferred second test strip (3), as shown in Figure 4C, comprises
- a backing layer,
- a sample pad as sample application site (31),
- the zone comprising protein-conjugates (35) comprises anti-p24 (clone 1) gold conjugate,
- test zone (32) comprising immobilized anti-p24 (clone 2),
- a control line with immobilized non-specific antibody as control zone (33),
- the material of the test strip in the areas of zones 32 and 33 is a nitrocellulose membrane,
- the remaining material of the test strip is an absorbing pad and a blood filter, and
- a pressure sensitive adhesive to assemble/connect the sample pad, nitrocellulose membrane and absorbing pad to the backing layer.

Furthermore, the test strips (2, 3) can comprise fiber glass pads containing the respective gold protein-conjugates which will be released when liquid, such as the sample, flows through the fiber glass pad.

Especially the detection of the HIV antigen p24 will not be efficient without signal enhancement and/or signal amplification because this antigen is available in HIV infected serum/plasma within the range of pg/ml; while the sensitivity of conventional rapid immunochromatographic tests that are known in the art lies in the range of only ng/ml. Thus, in order to be able to detect p24 antigen in early stages of HIV infection an ultra sensitive assay or device that allows for the detection of 10 pg/ml or lower has to be used, and the signals have to be enhanced and/or amplified.

According to the invention the gold protein conjugates in the zone (25, 35) are modified with a compound selected from the group comprising chitosan, oligochitosan, glucosamine and polylysine.

The action of any of these compounds or mixtures thereof is on the color intensity of the colloidal gold. They are added during the preparation of colloidal gold, but before the conjugation of colloidal gold with protein, i.e. antibody or antigen. The colloidal gold is conjugated after the modification with chitosan (and/or other modifiers) with a specific antibody and/or an antigen. The chitosan and the other modifiers affect the color intensity of colloidal gold and so increase the ability of the human eye to identify the color, and, thus, enable to detect very low concentrations. Signal amplification lies in the range of up to 10fold

It is preferred to prepare the colloidal gold by the reduction of 1% aqueous solution of tetrachloroauric acid (HAuC14) using trisodium citrate aqueous solution to produce spheroid gold particles. After colloidal gold preparation, chitosan (or any other modifier or mixture) aqueous solution was added with a suitable volume and concentration to convert the color from purple to violet depending on the volume and concentration of the added modification solution.

It is advantageous to combine the above described signal amplification with another signal enhancement method to further improve the sensitivity of the device. This can be carried out by either an internal signal enhancement, i.e. the device already comprises the required enhancement reagent(s), or an external signal enhancement, i.e. the reagents are added later to the device after the sample has already been applied.

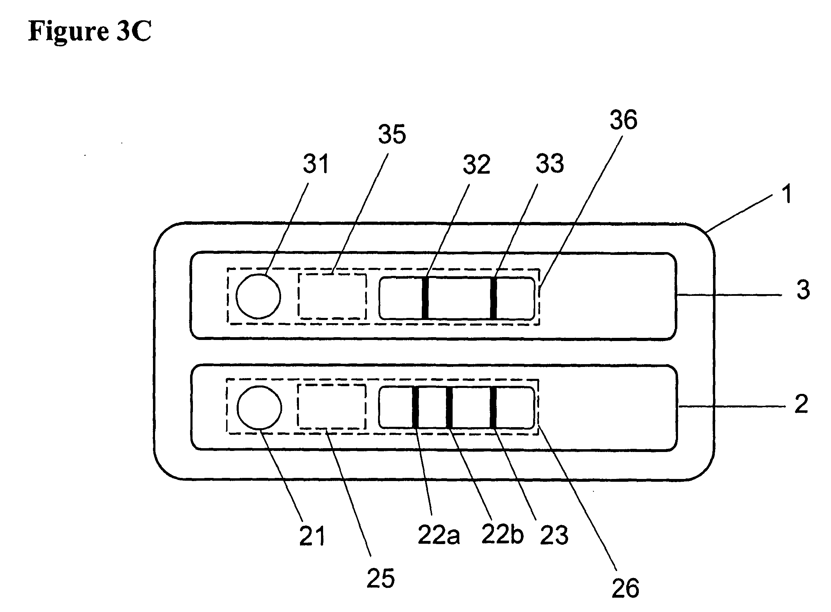

There is a preferred device according to the invention, wherein one or both of the two test strips further comprise a zone comprising signal enhancement compounds (26, 36) (for an embodiment see Figure 3C).

Silver reagents can be utilized for signal enhancement, wherein the gold particles will nucleate silver deposition, also called immunogold-silver staining method (IGSS). Metal particles can nucleate the highly specific deposition of silver from an appropriate silver salt solution in the presence of a suitable reducing agent. The silver coated gold particle then catalyzes more silver deposition and the silver grains grow in size. In this way even small gold particles may be enlarged up to 30 to 100 nm in size, resulting in greatly enhanced visibility.

Thus, in a preferred embodiment internal silver enhancement is utilized. Therefore, said zone (26, 36) comprises one or more silver reagents. The achieved signal enhancement by using a silver reagent or a mixture of silver reagents is in the range of 10fold to 50fold.

Furthermore, the reducing agent and the silver reagent(s) can be comprised in the device in said zone (26, 36), e.g. as separated dried spot(s)/line(s), or can be applied as external reagent(s).

Therefore, by combining silver enhancement with another signal amplification, such as modification of gold by chitosan and/or other modifiers (see above), the final signal increase can be in the range of 100fold to 500fold. As a result, ultra low concentrations in the range of 10 pg/ml or lower of p24 can be detected in the second test strip of the device according to the invention, which allows to detect p24 in early stages of an HIV infection.

Preferred silver reagents are silver acetate and hydroquinone. It, is preferred to use silver acetate and hydroquinone in citrate buffer or an immediate mixture of both. The citrate buffer is composed of citric acid monohydrate and sodium citrate. Also a mixture of all aforementioned silver reagents can be utilized.

Preferably respective dried components of the silver reagent(s) are applied onto the test strips. The reagent(s) will be released when the sample flows through the test strip or the respective zones of the test strip.

Furthermore, it is preferred to apply different silver enhancement reagents, preferably two different silver reagents, such as silver acetate in citrate buffer reagent and hydroquinone in citrate buffer reagent. It is possible to apply the different silver reagents in the same zone (26, 36). Thus, in one embodiment said zone (26, 36) comprises one or more silver reagents.

However, it is preferred to separate the different silver reagents, i.e. apply the different reagents at different sites on the test strip. Then, the test strips would comprise more than one zone containing the silver reagents. In one embodiment one of the silver reagents is applied at or near the sample application site (21, 31), preferably right "behind" to the sample pad, i.e. between the sample pad and the test zone (22a, 32), and the other silver reagent is applied onto the nitrocellulose membrane of the test strip. According to this design of the test strips, firstly the dried silver salt/reagent is released and later the reducing agent will be released. Therefore, after releasing of silver ions they will be reduced by the reducing reagent to form silver ions which will enhance the signal.

Such silver enhancement methods, the respective reagents and their use in assay devices are also disclosed, for example, in US 6,146,589.

The zone containing the gold conjugates (25, 35) and the zone containing the silver reagent(s) (26, 36) can furthermore overlap, for an embodiment see Figure 3C.

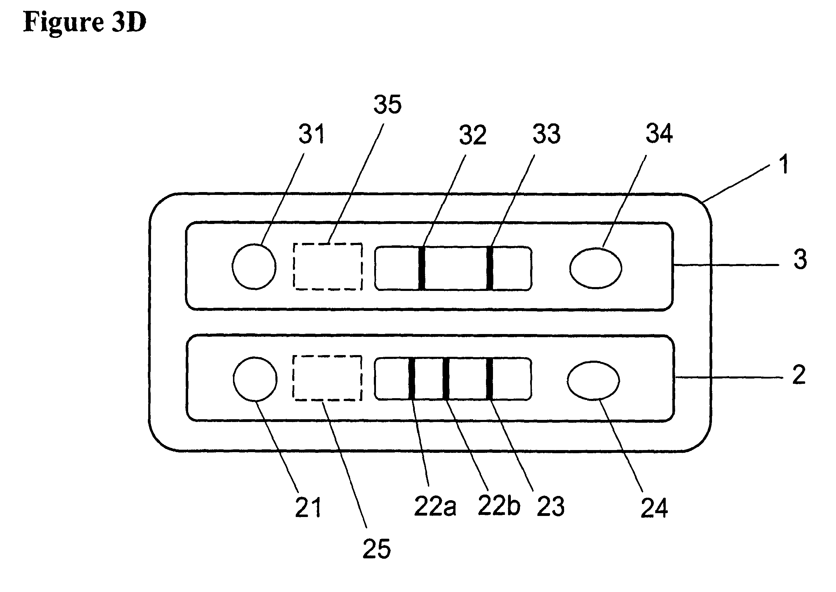

It is furthermore preferred that the test strips further comprise a reading zone (24, 34), for an embodiment see Figure 3D. The reading zones can simply show if the test strip in this zone is wet from the moving sample liquid. Thus, the reading zones can be used to determine if enough time has passed since applying a sample to the test strip.

The reading or validity zone (24, 34) can also comprise a humidity indicating colored material that will change its color in the presence of a specific relative humidity, so it acts as a validity indicator before usage to ensure that the device was s tored at suitable conditions (e.g. not humid conditions). The same zone (24, 34) can act as a reading zone during the usage of the device; i.e. its color will change when it reacts with water or vapor that originates from the strip flow, so the color will change when the sample reaches the reading zone, and so indicate the time for reading the results in the test and control zones. Also a third color could be comprised in the reading or validity zone (24, 34) to indicate that the time for reading results has finished.

It is preferred that a transparent material of the housing will allow for reading the results from the reading zones (24, 34) of each test strip (2, 3). The transparent materials of the housing can also have enclosures which can be opened at the time point of reading.

The problem is further solved by the present invention by providing uses of the test device according to the invention.

It is preferred to use the device of the invention for the early and rapid detection of HIV in a sample.

Samples, wherein HIV can be detected, can be bodily fluids like serum, plasma and whole blood, or any other fluid. They can also be supernatants from cultures that have been inoculated with cells from a patient suspected of being infected.

Furthermore, a method for the early and rapid detection of HIV in a sample using the device according to the invention is preferred.

Such a method preferably comprises the following steps:
- applying sample to the sample application site (21, 31) of each test strip (2, 3) in the device according to any of claims 1 to 6, and
- allowing the sample to move through the test zones (22a, 22b, 32) and control zones (23, 33) of the test strips (2, 3) of said device.

It is preferred to allow the sample to move through the test zones (22a, 22b, 32) and control zones (23, 33) of the test strips (2, 3) of said device until the color of the reading zones (24, 34) changes.

In one embodiment it is preferred to utilize external silver enhancement. Then the method for the early and rapid detection of HIV in a sample using the device according to the invention would comprise an additional step:
- treating with a silver reagent for signal enhancement.

As discussed above, treatment with a silver reagent or silver reagents is carried out for signal enhancement, wherein the gold particles will nucleate silver deposition, also called immunogold-silver staining method (IGSS). Metal particles can nucleate the highly specific deposition of silver from an appropriate silver salt solution in the presence of a suitable reducing agent. The silver coated gold particle then catalyzes more silver deposition and the silver grains grow in size. In this way even small gold particles may be enlarged up to 30 to 100 nm in size, resulting in greatly enhanced visibility.

The addition of the silver enhancement reagent(s) is carried out after the preparation of gold conjugate by soaking and preparation of the test strip. The reagent(s) are added as a final step after a faint signal has formed, and by its addition the signal will be enhanced and appear more clearly.

The signal enhancement that can be achieved by using a silver reagent or a mixture of silver reagents is in the range of 10fold to 50fold.

Therefore, by combining silver enhancement with another signal amplification, such as modification of gold by chitosan and/or other modifiers (see above), the final signal increase can be in the range of 100fold to 500fold. As a result, ultra low concentrations in the range of 10 pg/ml or lower of p24 can be detected in the second test strip of the device according to the invention, which allows to detect p24 in early stages of an HIV infection.

Preferred silver reagents are silver acetate and hydroquinone. It is preferred to use silver acetate and hydroquinone in citrate buffer reagent or an immediate mixture of both. Also a mixture of all aforementioned silver reagents can be utilized.

Furthermore, a kit for the rapid detection of HIV in a sample that comprises the test device according to the invention is preferred.

Preferably, such a kit further comprises further reagents and wash buffers and a manual. In a preferred embodiment the kit comprises silver reagent(s). For applying the kit in serum/plasma or whole blood testing it is preferred that the kit comprises an additional buffer, an assay buffer, preferably phosphate or Tris buffer.

The present invention relates to a screening assay for detecting early human immunodeficiency virus (HIV) infection. The assay belongs to the 4^{th} generation HIV assays that allow for the combined detection of HIV antigen and HIV antibody (Ag/Ab).

The first test strip of the device according to the invention serves as HIV type 1 and 2 antibodies detection system, which allows the separate detection of HIV-1 and HIV-2 antibodies. The first test strip contains two test zones, wherein one of these two test zones contains HIV-1 antigen, such as HIV-1 synthetic or recombinant proteins, e.g. p24 or p41.

The other of the two test zones contains HIV-2 antigen, such as HIV-2 synthetic or recombinant proteins, e.g. p36. The control zone contains a non-specific capturing antibody, such as for mouse clone conjugates an anti-mouse antibody and for rabbit clone conjugates an anti-rabbit antibody. In this first test system a sandwich immunoassay is utilized. This assay format requires two antibodies/antigens that are capable of binding to discrete, non overlapping epitopes on the antigen/antibody. This matched pair of antibodies/antigens ideally should have a high combined affinity and specificity.

In contrast, the prior art system disclosed in CA 2,431,778 cannot determine whether the HIV infection is of type 2 or 1. Furthermore, the detection of HIV-1 and HIV-2 antibodies in the same test zone (as disclosed in CA 2,431,778) by a combination of HIV-1 and HIV-2 synthetic/recombinant antigens decreases the specificity of the test.

The second test strip serves as HIV protein 24 (p24) detection system. It contains one test zone. A sandwich immunoassay using a pair of monoclonal antibodies specific for p24 is used, wherein "a gold conjugate of the first clone was prepared; the other clone was used on the capture line". Most likely, one p24 antibody is attached to the test strip in the test zone (i.e. capture line) and serves as primary antibody, the other p24 antibody is gold conjugated and serves as secondary antibody. The signals are amplified by the use of chitosan and/or oligo chitosan and/or glucosamine and/or polylysine and/or other polymers. The signals are further enhanced by the use of silver reagent. The control zone of the second test strip contains a non-specific capturing antibody, such as for mouse clone conjugates an anti-mouse antibody and for rabbit clone conjugates an anti-rabbit antibody. As a result, the second test system is very sensitive, because ultra low concentrations in the range of 10 pg/ml or lower of p24 can be detected, which allows to detect p24 in early stages of an HIV infection.

In contrast, the prior art system disclosed in CA 2,431,778, uses only one test strip containing two test zones, one test zone for HIV 1+2 antibody detection and the other test zone for p24 antigen detection. Thus, most likely a mixture of two conjugates has to be used, namely anti-p24 conjugate and anti-human immunoglobulin conjugate. In such a case the serum/plasma sample contains a complex of p24 captured by human immunoglobulin. However, such a complex will be captured by the two conjugates at the same time and mostly none of the test zones will be able to capture the resultant complex, leading to a decrease in sensitivity and in accuracy of the test. The probability of interaction between the sample (serum or plasma) and the gold conjugate will be decreased due to the availability of two conjugates at the same time.

Furthermore, the housing (1) of the test device accommodates both test strip systems (2, 3). The openings and/or transparent material of the housing serve for receiving test samples and for reading the results from the test zones (22a, 22b, 32) and control zones (23, 33).

Rapid immunochromatographic tests are widely used for the rapid detection of HIV antibodies. These tests require no laboratory infrastructure or highly skilled personnel and require about 5 to 30 minutes for obtaining the result. Therefore, these tests can be used onsite for screening of an HIV infection, thereby facilitating the process in rural areas. Such rapid tests may be useful if the result is needed quickly, for instance in emergency rooms, before emergency operations, after needle-stick injuries and to minimize the rate of "unclaimed" test results (if the result is only available after a few days, some of those tested will not return to receive it). Rapid tests, which are easy to perform and require little in terms of equipment, are also useful in developing countries [17-19].

The rapid immunochromatographic p24 antigen test, which is realized in the second test strip (3) of the test device according to the invention, has the following advantages:
- Detection of early HIV infection (9.4 -17.4 days earlier).
- Detection within the diagnostic window of the anti-HIV rapid tests and, thus, reduction of the false negative cases within this window.
- Early treatment or inclusion in clinical trials designed to ascertain the long-term benefits of early intervention.
- Reduction of HIV transmission risk by blood donation.
- Diagnosis of HIV infection in newborns.
- Monitoring antiviral therapy.
- Reduction of diagnosis costs.
- Reduction of time for obtaining the result: to about 10 minutes.
- No requirement of cumbersome sample transport and pretreatment procedures, unlike for RNA measurement.
- Useful in developing countries.
- No need for laboratory infrastructure.
- No need for highly skilled personnel.
- Useful in detecting antigen in supernatants from cultures that have been inoculated with cells from a patient suspected of being infected.

Furthermore, the combination of HIV 1/2 antibodies and p24 antigen detection systems in the test device according to the invention has the following advantages:
- Modification of 4^{th} generation assays that could be available for all.
- Improvement of p24 antigen detection limit in 4^{th} generation assays (detection of p24 concentration of 10 pg/ml).
- Complete system for HIV screening.
- Could be modified to be a semi-quantitative rapid test, i.e. determine the stage of HIV infection.

### References

1. Zaaijer, H.L., Exel-Oehlers, P.V., Kraaijeveld, T., Altena, E., Lelie, P.N. (1992) Early detection of antibodies to HIV-1 by third-generation assays. Lancet 340, 770-772.
2. Constantine, N.T., van der Groen, G., Belsey, E.M., Tamashiro, H. (1994) Sensitivity of HIV-antibody assays determined by seroconversion panels. AIDS 8, 1715-1720.
3. Satten, G.A., Busch, M.P., et al. (1997) Effect of transmission route on window period estimates. Fourth Conference on Retroviruses and Opportunistic Infections, Washington DC, Abstract 122.
4. WHO. Rapid HIV tests: Guidelines for use in HIV testing and counseling services in resource-constrained settings. Geneva 2004. http://www.who.int/hiv/pub/vct/rapidhivtests/en/
5. Holodniy M, et al. (1991) Reduction in plasma human immunodeficiency virus ribonucleic acid following dideoxynucleoside therapy as determined by the polymerase chain reaction. J. Clin. Invest 88, 1755-1759.
6. Katzenstein D.A., et al. (1994) Quantitation of human immunodeficiency virus by culture and polymerase chain reaction in response to didanosine after long-term therapy with zidovudine. J. Infect. Dis. 169, 416-419.
7. Jackson JB, et al. (1998) Practical diagnostic testing for human immunodeficiency virus. Clin. Microb. Rev. 1, 124-138.
8. Goudsmit J, et al. (1986) Expression of human Immunodeficiency virus antigen (HIV-Ag) in serum and cerebrospinal fluid during acute and chronic infection. Lancet 2, 177-180.
9. Aubuchon, J.P., Birkmeyer, J.D., Busch, M.P. (1997) Cost-effectiveness of expanded human immunodeficiency virus-testing protocols for donated blood. Transfusion 45, 45-51.
10. Ward, J.M., Holmberg, S.D., Allen, J.R., Cohn, D.L., et al. (1988) Transmission of human immunodeficiency virus (HIV) by blood transfusion screened as negative for HIV antibody. N. Engl. J. Med. 8, 473-478.
11. Alter, H.J., et al. (1990) Prevalence of human immunodeficiency virus type 1 p24 antigen in U.S. blood donors - an assessment of the efficacy of testing in donor screening. N. Engl. J. Med. 323, 1312-1317.
12. Mayers, D.L. (1998) Drug-resistant HIV-1. The virus strikes back. JAMA 279, 2000-2002.
13. Stephenson, J. (2002) Cheaper HIV drugs for poor nations bring a new challenge: monitoring treatment. JAMA 288, 2.
14. WHO. HIV assays: Operational characteristics (Phase 1). Report 15/ antigen/antibody ELISAs. Geneva 2004. http://www.who.int/diagnostics laboratory/evaluations/hiv/en/
15. WHO. HIV assays: Operational characteristics (Phase 1). Report 14/ simple/rapid tests. Geneva 2003. http://www.who.int/diagnostics_laboratory/evaluations/hiv/en/
16. Meier, T, et al. (2001) Evidence for a diagnostic window in fourth generation assays for HIV. J. Clin. Virol. 23, 113-116.
17. Aidoo, S., et al. (2001) Suitability of a Rapid Immunochromatographic test for detection of antibodies to human immunodeficiency virus in Ghana, West Africa. J. Clin. Microbiol. 2572-2575.
18. Mundee, Y., Kamtorn, N., et al. (1995) Infectious disease markers in blood donors in northern Thailand. Transfusion 35, 264-267.
19. Branson BM. (2003) Patientennahe Schnelltests für den Nachweis von HIV-Antikörpern. Point-of-Care Rapid Tests for HIV Antibodies. J. Lab. Med. 27, 288-295.

### Brief Description of Drawings

### Figure 1 HIV infection profile.

Following HIV infection intense viral replication results in high levels of HIV ribonucleic acid (RNA) and HIV p24 protein in plasma. Increases in anti-HIV antibody are detected. A variable window period, also called diagnostic window 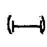 , is present between the time of infection and the point at which the infection can be detected. HIV RNA levels are detected earliest, followed by HIV p24 antigen and, finally, HIV antibody production.

### Figure 2 Principle of the 4^{th} generation HIV assays.

4^{th} generation HIV assays allow the combined detection of HIV antibodies and HIV antigen p24. Thereby, the detection of the HIV antibodies is based on the principle of double antigen i.e. also the early IgM antibodies are detected. In a preferred embodiment of the device according to the invention colloidal gold protein-conjugates are utilized.

### Figure 3 Design of the test device for the rapid detection of HIV according to the invention.

The test device comprises a housing (1) which accommodates two rapid immunochromatographic tests in form of two test strips (2, 3) which both will work together as a full screening system. The housing further comprises suitable openings and transparent materials.
**A** Each test strip comprises at least one sample application site (21, 31), at least one test zone and one control zone (23, 33). The first test strip (2) comprises a first test zone (22a) which comprises HIV-1 antigen(s) and a second test zone (22b) which comprises HIV-2 antigen(s). The second test strip (3) comprises a third test zone (32) which comprises anti-p24 antibody. Transparent materials (12, 13) of the housing (1) allow for reading the results from the test zones (22a. 22b, 32) and control zones (23, 33).
   The test strips (2, 3) are shown to be arranged in parallel such that the at least one sample application site (21, 31), at least one test zone (22a, 22b, 32) and one control zone (23, 33) of each test strip are in parallel as well.
**B** In a preferred embodiment, each test strip further comprises a zone comprising protein-conjugates (25, 35), wherein the protein-conjugates in one or both zones can comprise gold protein-conjugates.
**C** The test strips can comprise a zone comprising signal enhancement reagent(s), preferably silver reagent(s) (26, 36). However, it is also possible that the test strips comprise several zones comprising different silver reagents.
**D** The test strips can further comprise a validity or reading zone (24, 34).

### Figure 4 Preferred embodiments of the two test strips of the test device.

**A** A preferred embodiment of the test strips (2, 3) of the test device is shown.
   The test strips (2, 3) comprise a backing layer; a sample pad as sample application site (21, 31); a gold conjugate pad comprising gold protein-conjugates (25, 35); a capture line as test zone (22a, 32); a control line as control zone (23, 33); a nitrocellulose membrane as the material of the test strip in the areas of zones 22a, 32 and 23, 33; as remaining material of the test strip an absorbing pad, a blood filter and a pressure sensitive adhesive to connect the sample pad, nitrocellulose membrane, blood filter and absorbing pad to the backing layer.
**B** A preferred embodiment of the first test strip (2) of the test device is shown.
   The first test strip (2) comprises a backing layer; a sample pad as sample application site (21); anti-human immunoglobulins in the zone comprising protein-conjugates (25); test zones (22a, 22b) comprising immobilized HIV-1 antigen(s) or HIV-2 antigen, respectively; a control line with immobilized non-specific antibody as control zone (23); a nitrocellulose membrane as the material of the test strip in the areas of zones 22a, 22b and 23; as remaining material of the test strip an absorbing pad, a blood filter and a pressure sensitive adhesive to connect the sample pad, nitrocellulose membrane and absorbing pad to the backing layer.
**C** A preferred embodiment of the second test strip (3) test device is shown.
   The second test strip (3) comprises a backing layer; a sample pad as sample application site (31); anti-p24 (clone 1) gold conjugate in the zone comprising protein-conjugates (35); the test zone (32) comprising immobilized anti-p24 (clone 2); a control line with immobilized non-specific antibody as control zone (33); a nitrocellulose membrane as the material of the test strip in the areas of zones 32 and 33; as remaining material of the test strip an absorbing pad, a blood filter and a pressure sensitive adhesive to connect the sample pad, nitrocellulose membrane and absorbing pad to the backing layer.

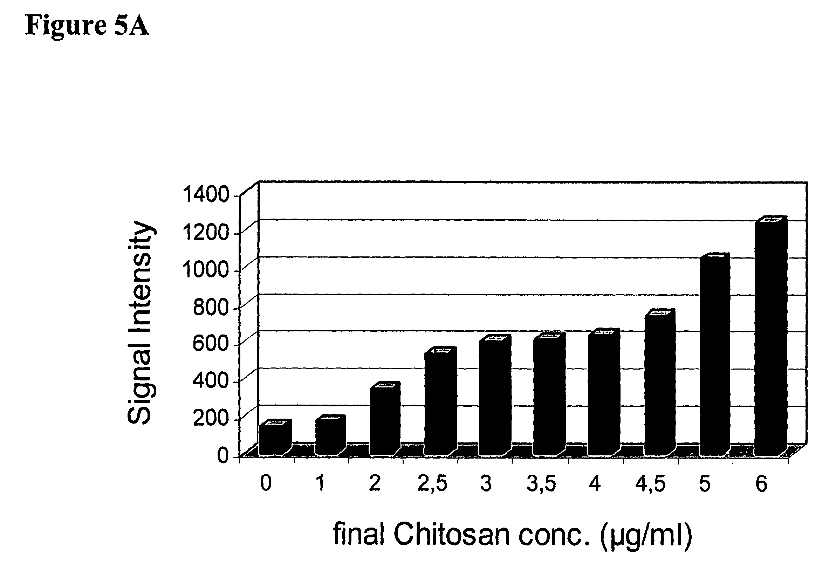

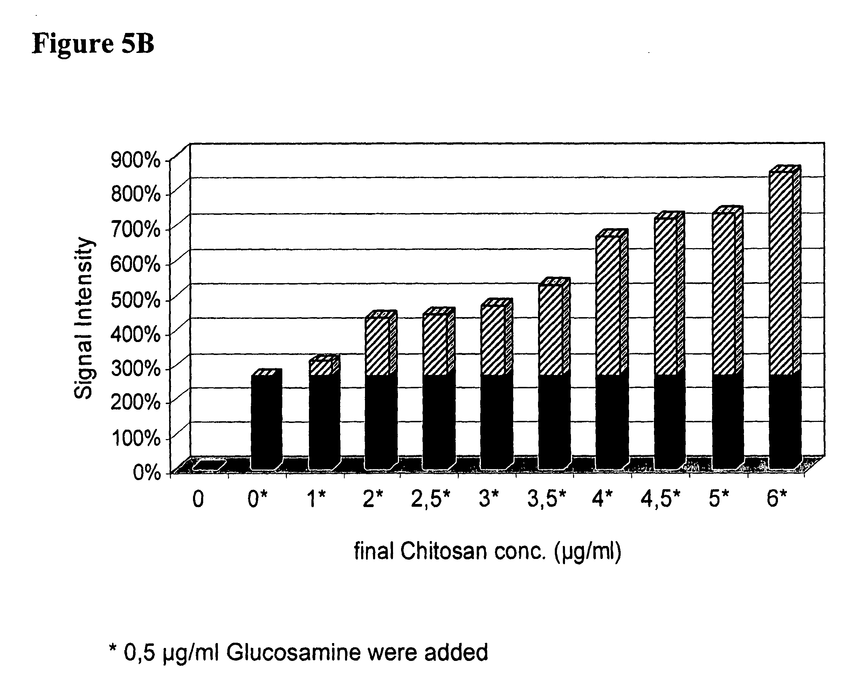

### Figure 5 Signal amplification by chitosan treatment.

**A** Second test strips were prepared using colloidal gold solutions that were treated with different concentrations of chitosan, as described in Example 1. These second test strips were compared in their ability to detect p 24 HIV antigen. An almost 8fold amplification of the signal was reached with the highest concentration of chitosan used, i.e. 6.0 µg/ml.
**B** Further second test strips were prepared using colloidal gold solutions that were treated with different concentrations of chitosan in the presence of a fixed amount of glucosamine (0.5 µg/ml), as described in Example 1. These second test strips were also compared in their ability to detect p24 HIV antigen. Thereby, treatment with glucosamine alone amplified the signal 2.5fold. An 8.5fold amplification of the signal was reached after treatment with 0.5 µg/ml glucosamine and 6.0 µg/ml chitosan together.

### Examples

The following examples are intended for illustration purposes only, and should not be construed as limiting the scope of the present invention in any way.

### Example 1: Preparation of gold conjugates

### a, Modification of colloidal gold with chitosan

Colloidal gold was prepared by the reduction of an 1% aqueous solution of tetrachloroauric acid (HAuCl₄) using 4% trisodium citrate aqueous solution to produce spheroid gold particles. After colloidal g old preparation, an aqueous solution of chitosan (or any other modifier or mixture) was added with a suitable volume and concentration to convert the color of the gold particle solution from purple to violet depending on the volume and concentration of the added modification solution. The color of the resultant colloidal gold solutions were turned to violet depending on the quantity of developer used. Chitosan, oligochitosan, glucosamine, polylysine or other polymers or mixtures thereof are suitable developers. The reagents used were prepared in house.

### b, Conjugation with protein

Monoclonal anti-p24 antibody was diluted in phosphate buffer before the addition of colloidal gold that was prepared as described above. Time was given for the conjugation to complete, typically between 30-60 minutes, before stabilizing buffers were added that contain polyvinylpyrrolidone (PVP), bovine serum albumin (BSA), Tween20, and sucrose in a phosphate buffered media with sodium azide as a preservative. Resultant conjugates were of colloidal gold with a color that is turned to violet depending on the quantity of developer used.
Furthermore, polyclonal anti-p24 antibody is suitable as well. Other suitable buffers are Tris, borate or others.

After conjugate preparation, the solution was centrifuged using a high-speed centrifuge to collect the pellet. The pellet was resuspended using phosphate buffer containing polyvinylpyrrolidone (PVP), bovine serum albumin (BSA), Tween20, and sucrose and soaked onto diagnostic grade fiber sheets. (Instead of phosphate Tris, borate or other buffers can be used.) The sheets then contain the conjugate and are used to prepare second test strips (3) of the device. These second test strips can be obtained from a so called p24 detection card. The preparation of such a p24 detection card is carried out by laminating the respective gold releasing pads, sample pads, absorbent pad, and monoclonal anti-p24 printed nitrocellulose membrane on a lateral backing. These cards are then cut into the respective second test strips (3) (see Figure 4C) and assembled into the plastic housing of the device for the detection of p24 antigen.

The same procedure is used for the preparation of the first test strip (2) for HIV-1 and HIV-2 antibodies; the only difference is that different antibodies/antigens are used for this strip (as can be seen in Figure 4B).

### Example 2: Signal amplification and signal enhancement

### a, Signal amplification by Chitosan treatment

Second test strips were prepared using colloidal gold solutions that were treated with different concentrations of chitosan, as described in Example 1. These second test strips were compared relatively in their ability to detect p24 HIV antigen using a density-scanning device, the Bio-Rad Gel Doc System. The results are shown in Table 1 and Figure 5A. An almost 8fold amplification of the signal was reached with the highest final concentration of chitosan used, i.e. 6.0 µg/ml final concentration in the gold solution.

**Table 1**

| Chitosan Concentration (µg/ml) | Signal Intensity | % Signal | Signal Enhancement |
|---|---|---|---|
| 0.0 | 140 | 100.00% | 0.0% |
| 1.0 | 169 | 120.71% | 20.71% |
| 2.0 | 344 | 245.71% | 145.71% |
| 2.5 | 531 | 379.29% | 279.29% |
| 3.0 | 594 | 424.29% | 324.29% |
| 3.5 | 604 | 431.43% | 331.43% |
| 4.0 | 627 | 447.86% | 347.86% |
| 5.4 | 721 | 515.00% | 415.00% |
| 5.0 | 1025 | 732.14% | 632.14% |
| 6.0 | 1222 | 872.86% | 772.86% |

Further second test strips were prepared using colloidal gold solutions that were treated with different concentrations of chitosan in the presence of a fixed amount of glucosamine, as described in Example 1. These second test strips were compared in their ability to detect p24 HIV antigen. The results are shown in Table 2 and Figure 5B. The treatment with glucosamine alone amplified the signal 2.5fold. An 8.5 fold amplification of the signal was reached after treatment with 0.5 ug/ml glucosamine and 6.0 µg/ml chitosan together (concentrations are the final concentrations in the gold solution) using the Bio-Rad Gel Doc System.

**Table 2**

| Glucosamine Concentration (µg/ml) | Chitosan Concentration (µg/ml) | Signal Intensity | % Signal | Glucosamine Enhancement | Chitosan Enhancement |
|---|---|---|---|---|---|
| 0 | 0.0 | 75.3 | 100.00% | 0.00% | 0.00% |
| 0.5 | 0.0 | 277.7 | 368.58% | 268.58% | 0.00% |
| 0.5 | 1.0 | 308.3 | 409.29% | 268.58% | 40.71% |
| 0.5 | 2.0 | 403.0 | 534.96% | 268.58% | 166.37% |
| 0.5 | 2.5 | 410.7 | 545.13% | 268.58% | 176.55% |
| 0.5 | 3.0 | 428.3 | 568.58% | 268.58% | 200.00% |
| 0.5 | 3.5 | 472.3 | 626.99% | 268.58% | 258.41% |
| 0.5 | 4.0 | 577.0 | 765.93% | 268.58% | 397.35% |
| 0.5 | 4.5 | 616.3 | 818.14% | 268.58% | 449.56% |
| 0.5 | 5.0 | 625.7 | 830.53% | 268.58% | 461.95% |
| 0.5 | 6.0 | 714.3 | 948.23% | 268.58% | 579.65% |

### b, Signal enhancement by silver treatment

Signal enhancement by silver reagent was tested using above modified second test strips and the signal enhancement measured was within 10 to 50folds. This silver enhancement was carried out as disclosed in e.g. EP 0 808 457 B1 and corresponding US 6,146,589 of the British Biocell International Limited, Cardiff, UK (BBInternational Ltd.).

In summary, by using second test strips that were treated with chitosan, glucosamine, and silver reagent, as described above, the detection limit for p24 HIV antigen reached about 2.6 pg/ml.

Furthermore, different analytes other than p24 were tested, all results were reproducible.

## Claims

1. A test device for the early and rapid detection of HIV in a sample,
**characterized by**
a housing (1) comprising two test strips (2, 3), wherein each test strip comprises at least one sample application site (21, 31), at least one test zone (22a, 22b, 32) and one control zone (23, 33), wherein
a, the first test strip (2) comprises a first test zone (22a) comprising immobilized HIV-1 antigen and a second test zone (22b) comprising immobilized HIV-2 antigen, and
b, the second test strip (3) comprises a third test zone (32) comprising immobilized anti-p24 antibody,
wherein one or both of the two test strips further comprise, between the sample application site (21, 31) and the test zones (22a, 22b, 32), a zone (25, 35) comprising gold protein-conjugates, which are modified with a compound selected from chitosan, oligochitosan, glucosamine and polylysine.

2. The device according to claim 1, wherein said zone (25) is situated in said first test strip (2).

3. The device according to claim 2, wherein the gold protein-conjugates in said zone (25) are HIV-1 antigen gold conjugates and HIV-2 antigen gold conjugates or anti-human immunoglobulin gold conjugates.

4. The device according to claim 1, wherein said zone (35) is situated in said second test strip (3).

5. The device according to claim 4, wherein the gold protein-conjugates in said zone (35) are anti-p24 antibody gold conjugates.

6. The device according to any of the preceding claims, wherein the control zones (23, 33) comprise immobilized non-specific capturing antibody, such as anti-gold control antibody.

7. The device according to any of the preceding claims, wherein one or both of the two test strips further comprise a zone comprising signal enhancement compounds (26, 36).

8. The device according to claim 7, wherein said zone (26, 36) comprises one or more silver reagents.

9. The device according to any of the preceding claims, wherein the test strips further comprise a reading zone (24, 34).

10. A method for the early and rapid detection of HIV in a sample using the device according to any of claims 1 to 9.

11. The method according to claim 10, comprising the following steps of
applying sample to the sample application site (21, 31) of each test strip (2, 3) in the device according to any of claims 1 to 9,
allowing the sample to move through the test zones (22a, 22b, 32) and control zones (23, 33) of the test strips (2, 3) of said device, and
optionally treating with a silver reagent for signal enhancement.

12. A kit for the rapid detection of HIV in a sample, comprising the device according to any of claims 1 to 9.

13. The kit according to claim 12, further comprising further reagents and wash buffers and a manual.

14. The kit according to claim 12 or 13, comprising a silver reagent.

## Patentansprüche

1. Testvorrichtung zum frühen und schnellen Nachweis von HIV in einer Probe, **gekennzeichnet durch**
ein Gehäuse (1), das zwei Teststreifen (2, 3) umfasst, wobei jeder Teststreifen wenigstens eine Probenauftragsstelle (21, 31), wenigstens eine Testzone (22a, 22b, 32) und eine Kontrollzone (23, 33) umfasst, wobei
a, der erste Teststreifen (2) eine erste Testzone (22a) umfasst, die immobilisiertes HIV-1-Antigen umfasst, und eine zweite Testzone (22b), die immobilisiertes HIV-2-Antigen umfasst, und
b, der zweite Teststreifen (3) eine dritte Testzone (32) umfasst, die immobilisierten anti-p24-Antikörper umfasst,
wobei einer oder beide der zwei Teststreifen außerdem, zwischen der Probenauftragsstelle (21, 31) und den Testzonen (22a, 22b, 32), eine Zone (25, 35) umfasst, die Gold-Protein-Konjugate umfasst, die mit einer Verbindung modifiziert sind, die ausgewählt ist aus Chitosan, Oligochitosan, Glucosamin und Polylysin.

2. Vorrichtung nach Anspruch 1, wobei die Zone (25) im ersten Teststreifen (2) angeordnet ist.

3. Vorrichtung nach Anspruch 2, wobei die Gold-Protein-Konjugate in der Zone (25) HIV-1-Antigen-Gold-Konjugate und HIV-2-Antigen-Gold-Konjugate oder anti-Humanimmunglobulin-Gold-Konjugate sind.

4. Vorrichtung nach Anspruch 1, wobei die Zone (35) im zweiten Teststreifen (3) angeordnet ist.

5. Vorrichtung nach Anspruch 4, wobei die Gold-Protein-Konjugate in der Zone (35) anti-p24-Antikörper-Gold-Konjugate sind.

6. Vorrichtung nach einem der vorangehenden Ansprüche, wobei die Kontrollzonen (23, 33) immobilisierte nicht-spezifische Einfang-Antikörper, wie etwa anti-Gold-Kontroll-Antikörper, umfassen.

7. Vorrichtung nach einem der vorangehenden Ansprüche, wobei einer oder beide der zwei Teststreifen außerdem eine Zone umfassen, die Signalverstärkungsverbindungen (26, 36) umfassen.

8. Vorrichtung nach Anspruch 7, wobei die Zone (26, 36) ein oder mehrere Silberreagentien umfasst.

9. Vorrichtung nach einem der vorangehenden Ansprüche, wobei die Teststreifen außerdem eine Ablesezone (24, 34) umfassen.

10. Verfahren zum frühen und schnellen Nachweis von HIV in einer Probe durch Verwendung der Vorrichtung nach einem der Ansprüche 1 bis 9.

11. Verfahren nach Anspruch 10, umfassend die folgenden Schritte des
Aufbringens einer Probe auf die Probenauftragsstelle (21, 31) jeden Teststreifens (2, 3) in der Vorrichtung nach einem der Ansprüche 1 bis 9,
Ermöglichens, dass die Probe durch die Testzonen (22a, 22b, 32) und Kontrollzonen (23, 33) der Teststreifen (2, 3) der Vorrichtung hindurch wandert, und
gegebenenfalls Behandelns mit einem Silberreagens zur Signalverstärkung.

12. Kit zum schnellen Nachweis von HIV in einer Probe, der die Vorrichtung nach einem der Ansprüche 1 bis 9 umfasst.

13. Kit nach Anspruch 12, der weitere Reagentien und Waschpuffer und eine Anleitung umfasst.

14. Kit nach Anspruch 12 oder 13, der ein Silberreagens umfasst.

## Revendications

1. Dispositif de test pour la détection précoce et rapide du VIH dans un échantillon, **caractérisé par**
un boîtier (1) comprenant deux bandelettes de test (2, 3), où chaque bandelette de test comprend au moins un site d'application d'échantillon (21, 31), au moins une zone de test (22a, 22b, 32) et une zone de contrôle (23, 33), où
a, la première bandelette de test (2) comprend une première zone de test (22a) comprenant l'antigène du VIH-1 immobilisé et une deuxième zone de test (22b) comprenant l'antigène du VIH-2 immobilisé, et
b, la seconde bandelette de test (3) comprend une troisième zone de test (32) comprenant un anticorps anti-p24 immobilisé,
où l'une ou les deux des deux bandelettes de test comprennent en outre, entre le site d'application d'échantillon (21, 31) et les zones de test (22a, 22b, 32), une zone (25, 35) comprenant des conjugués or-protéine, qui sont modifiés avec un composé choisi parmi le chitosane, l'oligochitosane, la glucosamine et la polylysine.

2. Dispositif selon la revendication 1, dans lequel ladite zone (25) est située dans ladite première bandelette de test (2).

3. Dispositif selon la revendication 2, dans lequel les conjugués or-protéine dans ladite zone (25) sont des conjugués antigène du VIH-1-or et des conjugués antigène du VIH-2-or ou des conjugués immunoglobuline anti-humaine-or.

4. Dispositif selon la revendication 1, dans lequel ladite zone (35) est située dans ladite seconde bandelette de test (3).

5. Dispositif selon la revendication 4, dans lequel les conjugués or-protéine dans ladite zone (35) sont des conjugués anticorps anti-p24-or.

6. Dispositif selon l'une quelconque des revendications précédentes, dans lequel les zones de contrôle (23, 33) comprennent un anticorps de capture non spécifique immobilisé, tel qu'un anticorps de contrôle anti-or.

7. Dispositif selon l'une quelconque des revendications précédentes, dans lequel l'une ou les deux des deux bandelettes de test comprennent en outre une zone comprenant des composés d'amplification du signal (26, 36).

8. Dispositif selon la revendication 7, dans lequel ladite zone (26, 36) comprend un ou plusieurs réactifs d'argent.

9. Dispositif selon l'une quelconque des revendications précédentes, dans lequel les bandelettes de test comprennent en outre une zone de lecture (24, 34).

10. Procédé permettant la détection précoce et rapide du VIH dans un échantillon en utilisant le dispositif selon l'une quelconque des revendications 1 à 9.

11. Procédé selon la revendication 10, comprenant les étapes suivantes consistant à :
appliquer l'échantillon sur le site d'application d'échantillon (21, 31) de chaque bandelette de test (2, 3) dans le dispositif selon l'une quelconque des revendications 1 à 9,
laisser l'échantillon se déplacer le long des zones de test (22a, 22b, 32) et des zones de contrôle (23, 33) des bandelettes de test (2, 3) dudit dispositif, et
éventuellement traiter avec un réactif d'argent pour l'amplification du signal.

12. Kit pour la détection rapide du VIH dans un échantillon, comprenant le dispositif selon l'une quelconque des revendications 1 à 9.

13. Kit selon la revendication 12, comprenant en outre d'autres réactifs et des tampons de lavage et un manuel.

14. Kit selon la revendication 12 ou 13, comprenant un réactif d'argent.
